(19) Europäisches Patentamt
European Patent Office
Office européen des brevets

(11) **EP 1 450 665 B1**

(12) **EUROPÄISCHE PATENTSCHRIFT**

(45) Veröffentlichungstag und Bekanntmachung des Hinweises auf die Patenterteilung:
**17.05.2006 Patentblatt 2006/20**

(21) Anmeldenummer: **02790264.2**

(22) Anmeldetag: **27.11.2002**

(51) Int Cl.:
***A61B 1/227*** *(2006.01)*

(86) Internationale Anmeldenummer:
**PCT/DE2002/004342**

(87) Internationale Veröffentlichungsnummer:
**WO 2003/047415 (12.06.2003 Gazette 2003/24)**

(54) **OTOSKOP**

OTOSCOPE

OTOSCOPE

(84) Benannte Vertragsstaaten:
**AT BE BG CH CY CZ DE DK EE ES FI FR GB GR IE IT LI LU MC NL PT SE SK TR**

(30) Priorität: **27.11.2001 DE 20119187 U**

(43) Veröffentlichungstag der Anmeldung:
**01.09.2004 Patentblatt 2004/36**

(73) Patentinhaber: **Witte & Sutor GmbH**
**71540 Murrhardt (DE)**

(72) Erfinder: **WITTE, Waldemar**
**55120 Mainz (DE)**

(74) Vertreter: **Geitz, Holger**
**Geitz Truckenmüller Lucht**
**Patentanwälte,**
**Kriegsstrasse 234**
**76135 Karlsruhe (DE)**

(56) Entgegenhaltungen:
| | |
|---|---|
| **WO-A-00/41616** | **WO-A-00/51487** |
| **WO-A-01/28407** | **WO-A-02/071930** |
| **DE-A- 19 950 899** | **US-A- 2 943 184** |
| **US-A- 4 652 093** | **US-B1- 6 186 944** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein Otoskop, vorzugsweise zur medizinischen Untersuchung des menschlichen Gehörgangs, mit einem Griffstück und einem Kopfstück, wobei das Kopfstück mit einem Ohrtrichter zur teilweisen Einführung in einen Gehörgang unter Einschluß eines optischen Kanals verbunden ist, der am Kopfstück auf der dem Ohrtrichter abgewandten Seite mit einer integrierten Lupe zur vergrößerten Betrachtung des Gehörgangs versehen ist, wobei dem optischen Kanal ein Leuchtmittel zur Ausleuchtung des Gehörgangs zugeordnet ist.

Ein derartiges Otoskop ist aus der internationalen Patentanmeldung WO 00/51487 bekannt. Das aus dieser internationalen Anmeldung vorbekannte Otoskop zeichnet sich vor allem dadurch aus, daß neben der Untersuchung des Gehörgangs gleichzeitig Medikamente in den Gehörgang eingespritzt werden können. Die Medikation des Gehörgangs kann dabei vorteilhafterweise erfolgen, ohne daß das Otoskop entfernt werden muß. Hierdurch kann die Medikation des Gehörgangs besser überwacht werden. Um sowohl den Gehörgang untersuchen zu können, als auch die Medikation durchführen zu können, ist es erforderlich, dem zum Einführen in den Gehörgang dienenden Ohrtrichter mit einem Leuchtmittel zu versehen. Im Fall der vorbekannten Erfindung handelt es sich dabei um eine batteriegetriebene Glühlampe, die mit einem Lichtleiter verbunden ist, um das Licht an der gewünschten Stelle innerhalb des Ohrtrichters emittieren zu können. Der Lichtleiter bietet gegenüber der Glühlampe den Vorteil einer leichteren Positionierung und insbesondere der Lichtumlenkung innerhalb des Ohrtrichters.

Das zur Durchführung der Ohrenspiegelung seit vielen Jahrzehnten bekannte Otoskop - auch Ohrenspiegel oder Ohrenspekulum genannt - gewährleistet nur dann eine optimale Untersuchung des Gehörgangs, wenn der Gehörgang optimal ausgeleuchtet ist. Dabei hat es sich als nachteilig erwiesen, daß die Leuchtmittel schon nach wenigen Betriebsstunden aufgrund der nachlassenden Batteriespannung der zur Versorgung der Leuchtmittel verwendeten Batterien nur noch einen Bruchteil ihrer maximalen Leuchtkraft aufweisen. Der behandelnde Arzt muß demnach ständig die Batterien des Otoskops wechseln, um eine jeweils optimale Ausleuchtung des Gehörgangs sicher zu stellen. Dabei müssen die Batterien zum Teil auch nur zur Hälfte entladen entsorgt werden. Dies ist schon allein aus Umweltschutzgründen kaum akzeptabel.

Der Erfindung liegt daher die Aufgabe zugrunde, ein Otoskop zu schaffen, das die vorstehend erläuterten Nachteile vermeidet und zumindest für einen längeren Zeitraum eine optimale Ausleuchtung des Gehörgangs zum Zwecke der Untersuchung ermöglicht.

Die erfindungsgemäße Aufgabe wird gemäß den Merkmalen des Hauptanspruchs dadurch gelöst, daß dem Ohrtrichter als Leuchtmittel eine ,vorzugsweise weiße, Leuchtdiode zugeordnet wird.

Eine Leuchtdiode ist eine Halbleiterdiode, die beim Betrieb in Durchlaßrichtung Licht erzeugt. Dabei gibt ein Halbleiterkristall ein Lichtsignal ab, das durch die linsenförmige Form des Kopfes der Leuchtdiode gebündelt bzw. gestreut wird. Das mittels einer Leuchtdiode erzeugte Licht ist einfarbig, d.h. das Licht wird nur über einen schmalen Wellenlängenbereich abgestrahlt. Im Unterschied zu Glühlampen, bei denen ein Glühfaden zum Glühen gebracht wird, der dann Licht emittiert, wird bei Leuchtdioden mit dem erwähnten Halbleiterkristall nahezu die gesamte eingesetzte Energie in Licht umgewandelt. Bei Glühlampen werden über 90° der eingesetzten Energie in Wärme umgesetzt und abgestrahlt. Aufgrund einer nahezu vollständigen Umwandlung der Energie in Licht statt in Wärme, spricht man bei dem von einer Leuchtdiode erzeugten Licht von "cool light".

Die höhere Lichtausbeute der Leuchtdiode erhöht somit sofort die Lebensdauer der eingesetzten Batterien um ein Vielfaches. Leuchtdioden haben darüber hinaus den Vorteil, äußerst robust zu sein. Im Unterschied zum dünnen und empfindlichen Glühfaden einer Glühbirne ist die Leuchtdiode in eine Kunststofflinse eingebettet und deshalb ausgesprochen bruchsicher.

Die Lebensdauer von Leuchtdioden reicht im Dauerbetrieb mehrere Jahre.

Die Verwendung von Leuchtdioden im Bereich der Ohrenspiegelung gelingt vor allem dadurch, daß es vor wenigen Jahren gelungen ist, auch blaue Leuchtdioden herzustellen. Hierdurch ist es ermöglicht worden, im Rahmen der weiteren Entwicklung auch weiße Leuchtdiode zu produzieren. Durch die Kombination von blauen Galium-Nitrit-Leuchtdioden und bestimmten Farbstoffen ist es seit einigen Jahren gelungen, die für ein weißes Licht notwendige Mischung zu erzeugen.

Ein weiterer Vorteil der Leuchtdiode liegt in der Emission von diffusem Licht. Hierdurch wird vor allem im Nahbereich eine gleichmäßigere Ausleuchtung der angestrahlten Konturen erreicht.

Das erfindungsgemäße Otoskop erreicht bei Verwendung von weißen Leuchtdioden sowohl eine verbesserte Ausleuchtung des Gehörgangs wie aufgrund der höheren Energieeffizienz auch einen geringen Batterieverbrauch, so daß die verbesserte Ausleuchtung auch für einen langen Zeitraum anhält. Das Kopfstück ist mit dem Ladegriff des Otoskops gelenkig verbunden. Hierdurch ist je nach Patient und je nach Einführwinkel und -tiefe eine jeweils optimale Stellung des Otoskops ermöglicht.

Durch die Verwendung eines lösbaren Griffstücks kann im Falle des Nachlassens der Batterie oder Akkuspannung das Kopfstück einfach auf einen anderen Ladegriff aufgesetzt werden, um beispielsweise eine laufende Untersuchung fortzusetzen. Die lösbare Verbindung von Otoskop-Kopf und Ladegriff erfolgt idealerweise über einen einfachen Aufsteckadapter.

Dieser Adapter kann alternativ zum Otoskop-Kopf mit einem ansonsten herkömmlichen Taschenlampenkopf verbunden werden, um das Griffstück gegebenenfalls auch als Taschenlampe nutzen zu können. Umgekehrt ausgedrückt, könnte auch eine hinsichtlich der Stromführung entsprechend präparierte und mit einem Anschluß versehene, sonst aber herkömmliche Taschenlampe im Bedarfsfalle mit einem Otoskop-Kopf ausgerüstet werden. Hierdurch ist es möglich, ein Otoskop im Sinne einer Taschenlampe nachzurüsten und umgekehrt.

In vorteilhafter Ausgestaltung wird eine weiße Leuchtdiode eingesetzt, deren Lichtspektrum zumindest annähernd dem Spektrum des Sonnenlichts entspricht. Hierdurch ist eine naturgetreue Farbwiedergabe erreicht. Die korrekte Wiedergabe von Farben ist insbesondere bei der Diagnose des Gehörgangs von erheblichem Vorteil.

Die Batterien oder Akkus zum Betrieb der Leuchtdiode werden optimalerweise innerhalb des Griffstücks des Otoskops untergebracht und können an dieser Stelle leicht ausgewechselt werden.

Die Ausfallgefahr eines mit einer Leuchtdiode betriebenen Otoskops beschränkt sich im wesentlichen auf die Gefahr der Tiefentladung der verwendeten Akkus, sofern solche verwendet werdet. In diesem Fall ist im Rahmen der Erfindung die Schwellspannung der Leuchtdiode so bemessen ist, daß sie größer, gleich der Entladeschlußspannung des eingesetzten Stromspeichers ist, ist auch die Gefahr der Tiefentladung wirksam vermieden.

Für den Fall, daß die Leuchtdiode von einem Mehrzellenakku gespeist wird, sollte die Entladeschlußspannung der Akkuzellen und die Schwellspannung der Leuchtdiode unter Berücksichtigung der Zellenzahl aufeinander abgestimmt werden.

Im Interesse einer möglichst langen Betriebszeit des Otoskops werden idealerweise Nickel-Metall-Hydrid-Akkus eingesetzt.

In abermals vorteilhafter Ausgestaltung ist die Leuchtdiode derart im Ohrtrichter angeordnet, daß der an der Einführspitze aus der Mündung des Ohrtrichters austretende Lichtstrahl eine optimale Ausleuchtung des Gehörgangs gewährleistet.

Die Erfindung wird nachstehend anhand eines in der Zeichnung nur schematisch dargestellten Ausführungsbeispiels näher erläutert.

Es zeigen:

Fig. 1 ein Otoskop in einer Seitenansicht,

Fig. 2 eine Detailansicht zur Anordnung der Leuchtdiode innerhalb eines Ohrtrichters, des in Fig. 1 dargestellten Otoskops im Querschnitt und

Fig. 3 eine Detailansicht zur Anordnung einer Batterie innerhalb eines Ladegriffstücks, des in Fig. 1 dargestellten Otoskops im Querschnitt,

Fig. 4 eine Taschenlampe mit einem Adapterstück zum Aufsatz eines Otoskopkopfs in einer perspektivischen Darstellung,

Fig. 5 die in Fig. 4 gezeigte Taschenlampe mit abgezogener Ladehülse in einer perspektivischen Darstellung und

Fig. 6 die in den Fig. 4 und 5 gezeigte Taschen lampe mit aufgestecktem Otoskopkopf.

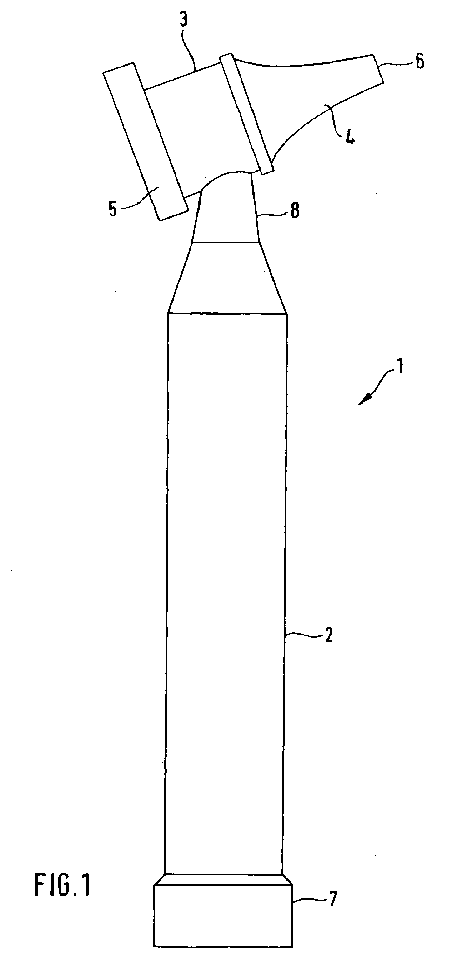

Das in Fig. 1 dargestellte Otoskop 1 besteht im wesentlichen aus einem Ladegriffstück 2, einem Kopfstück 3 und einem Ohrtrichter 4. Der Ohrtrichter 4 ist entweder mit dem Kopfstück 3 verschraubt oder im Wege einer Preßpassung aufgesteckt. In jedem Fall ist der Ohrtrichter 4 schon aus hygienischen Gründen lösbar mit dem Kopfstück 3 verbunden und kann ausgetauscht oder gereinigt werden. Das Kopfstück 3 weist an dem von dem Ohrtrichter 4 entfernten Ende eine integrierte Lupe 5 auf, die den Abschluß eines in Fig. 1 nicht weiter dargestellten optischen Kanals darstellt, der konzentrisch durch das im wesentlichen rotationssymmetrisch ausgebildete Kopfstück 3 und durch den ebenfalls im wesentlichen rotationssymmetrisch ausgebildeten Ohrtrichter 4 verläuft und schließlich an der Einführspitze 6 mündet. Die Einführspitze 6 wird bei bestimmungsgemäßem Gebrauch in den menschlichen Gehörgang eingeführt.

Das Kopfstück 3 ist gemäß der in Fig. 1 dargestellten Ausführung gelenkig mit dem Ladegriffstück 2 verbunden. Es kann allerding auch weitgehend unbeweglich auf dem Ladegriffstück 2 befestigt sein.

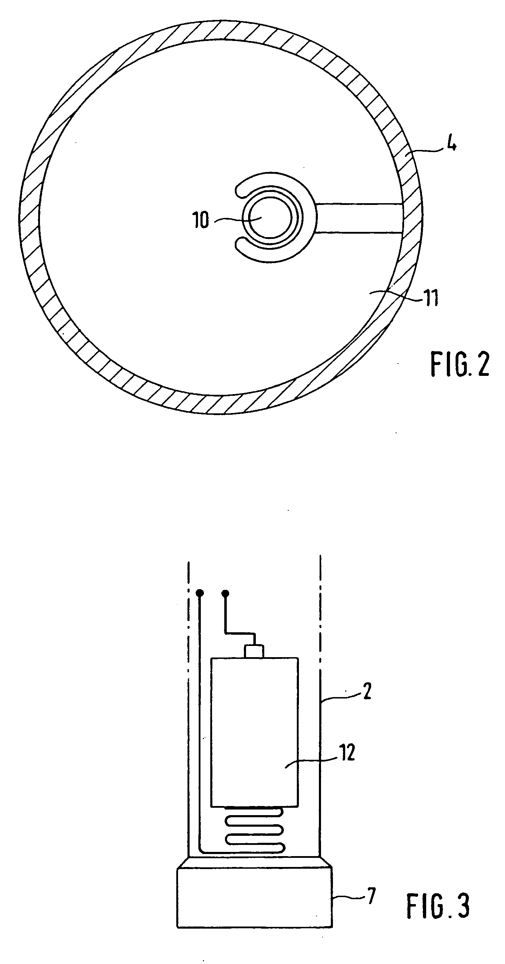

In der speziellen Ausführung nach Fig. 2 handelt es sich dabei um ein Kugelkopfgelenk, das auf einem Steckadapterstück 6 sitzt. Das Kopfstück 3 kann mitsamt dem Ohrtrichter 4 von dem Steckadapterstück 6 in einfacher Weise abgezogen werden und statt dessen beispielsweise ein Taschenlampenkopf ausgetauscht werden, dessen Strahlrichtung im wesentlichen in einer gedachten axialen Verlängerung des Ladegriffstücks 2 verläuft.

Das Ladegriffstück 2 ist an seinem von dem Kopfstück 3 abgewandten Ende mit einem Schraubverschluß 7 verschlossen und im wesentlichen als Hohlzylinder zur Aufnahme von einer oder mehrerer Batterien zur Energieversorgung eines Leuchtmittels ausgebildet.

Das von den im Ladegriffstück 2 austauschbar angeordneten Batterie versorgte Leuchtmittel 10 ist gemäß Fig. 2 derart im optischen Kanal des Ohrtrichters 4 angeordnet, daß sich bei bestimmungsgemäßem Gebrauch eine möglichst gleichmäßige Ausleuchtung des zu untersuchenden Gehörgangs ergibt. Bei dem Leuchtmittel handelt es sich um eine sogenannte weiße Leuchtdiode 10 (DED = light emittig diode), bei der blau emittierende Leuchtdioden auf Galium-Nitrid-Basis mit Luminizenzfarbstoffen zu einer Weißlicht-Diode kombiniert sind. Die weißen Leuchtdioden haben ein dem Sonnenlicht zumindest ähnliches Lichtspektrum und garantieren somit eine farbgetreue Wiedergabe. Leuchtdioden haben eine Lebensdauer von deutlich über zehn Jahren im Dauerbertrieb. Ersatzteile sind in dieser Zeit weitgehend überflüssig. Anstelle eines dünnen, im engsten Zustand besonders anfälligen Glühfadens, ist die Leuchtdiode in einer klaren Kunststofflinse eingebettet und gilt daher als extrem bruchsicher. Leuchtdioden setzen über 90 Prozent der eingesetzten Energie in Licht um. Der Strombedarf beträgt daher lediglich einen Bruchteil der ansonsten von Glühlampen für die gleiche Lichtleistung benötigte Energiemenge. Zusammengefaßt muß das Leuchtmittel während der üblichen Lebenszeit eines Otoskops nicht gewechselt werden.

Die gemäß Fig. 3 im Ladegriffstück 2 zur Stromversorgung der Leuchtdiode angeordneten Batterie 12, vorzugsweise eine herkömmliche Trockenbatterie, muß ebenfalls ausgesprochen selten erneuert werden.

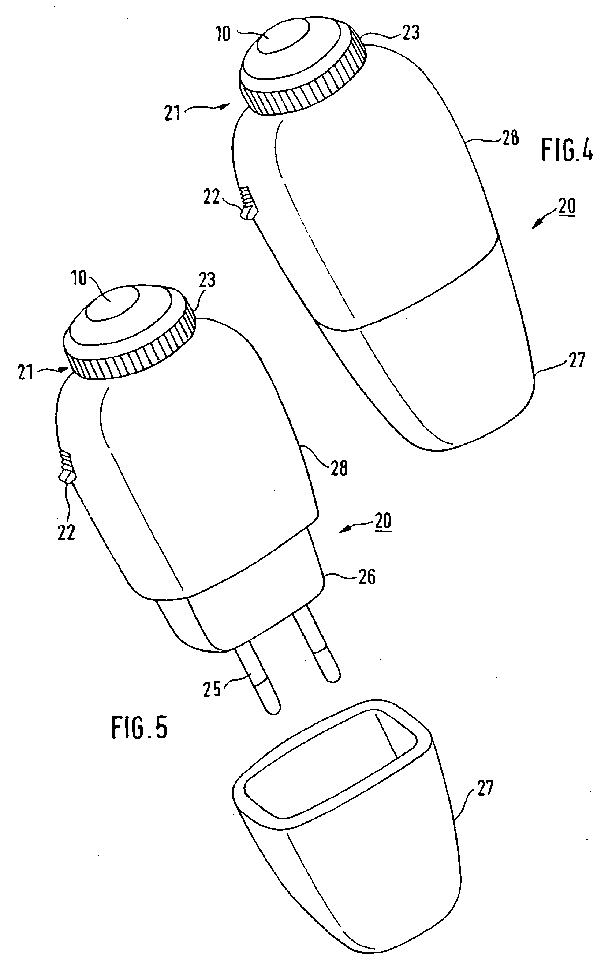

Gemäß einem anderen in den Fig. 4 bis 6 gezeigten Ausführungsbeispiel kann das Otoskop auch zunächst aus einer weitgehend herkömmlichen Taschenlampe 20, deren eine Stirnseite als Leuchtseite 21 mit einer weißen Leuchtdiode 10 die zentrisch innerhalb eines Schraubadapterstücks 23 angeordnet ist, aufgebaut sein. Die Taschenlampe 20 weist einen Mittelabschnitt 28 auf, in dem eine hier nicht weiter dargestellter Akku integriert ist. Der Akku wird über einen seitlich angeordneten Schiebeschalter 22 zur Herstellung eines Kontaktes mit dem Anschluß der Leuchtdiode 10 und/oder mit deren Fassung verbunden. Auf der von der Leuchtseite 21 abgewandten Seite ist der Mittelabschnitt 28 mit einem Ladeabschnitt verbunden. Der Ladeabschnitt besteht gemäß Fig.5 im wesentlichen aus einem Steckkontakt 25 zur bedarfsweisen Aufladung eines Akkus in einer hier nicht dargestellten Haushalts-Steckdose. Der Steckkontakt 25 ist gemäß Fig. 4 mittels einer auf einen Stutzen 26 des Steckkontaktes 25 formschlüssig aufschiebbare Steckhülse 27 abgedeckt.

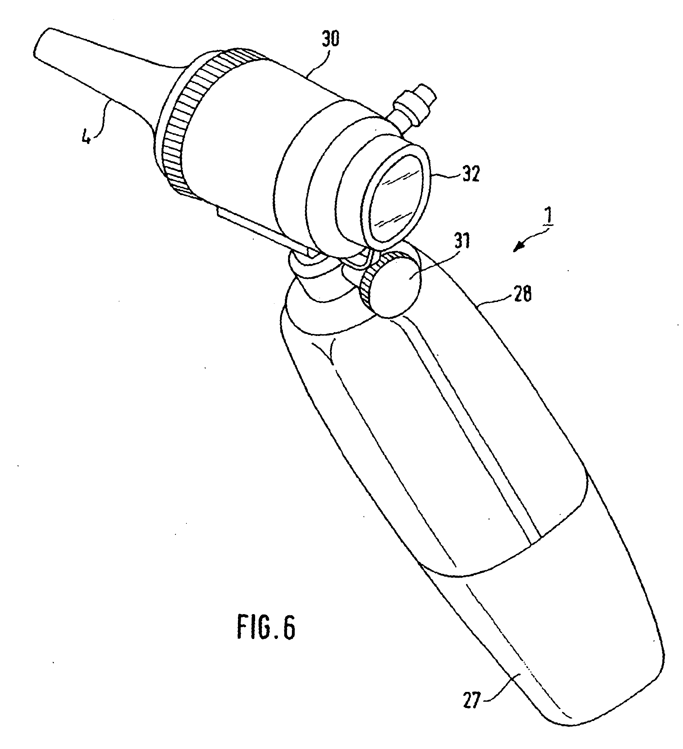

Gemäß der Darstellung in Figur 6 kann bedarfsweise auf der Leuchtseite der Taschenlampe 20 auf das Schraubadapterstück ein Otoskop -Kopfstück 30 mit einer weiteren integrierten weißen Leuchtdiode aufgesetzt werden. Das Otoskop-Kopfstück 30 weist ebenfalls einen Ohrtrichter 4 zur Einführung in den Gehörgang eines Patienten auf.

Die in der Zeichnung nicht weiter dargestellte zusätzliche weiße Leuchtdiode innerhalb des Otoskop-Kopfstücks 30 ist über den Schraubadapter elektrisch mit dem in dem Mittelabschnitt 28 integrierten Batterie verbunden. Dabei wird das Otoskop-Kopfstück 30 mit einer Feststellschraube 31 fest mit dem Schraubadapterstück verschraubt und somit gesichert.

Auf der dem Ohrtrichter 4 abgewandten Seite des Otoskop-Kopfstücks 30 ist eine verschwenkbar angelenkte Lupe 32 vorgesehen, die bedarfsweise in den im Gehörgang des Patienten mündenden Sichtkanal eingeschwenkt werden kann.

Das Otoskop-Kopfstück 30 stellt somit ein praktisches Zubehör für die in den Fig. 4 und 5 gezeigte Taschenlampe 20 dar.

Vorstehend ist somit ein Otoskop beschrieben, das ein gegenüber dem Stand der Technik überlegenes Leuchtmittel einsetzt und überdies einen geringeren Energiebedarf aufweist.

**Patentansprüche**

1.  Otoskop, vorzugsweise zur medizinischen Untersuchung des menschlichen Gehörgangs, mit einem Griffstück (2) und einem Kopfstück (3), wobei das Kopfstück (3) mit einem Ohrtrichter (4) zur teilweisen Einführung in einen Gehörgang unter Einschluß eines optischen Kanals (11) verbunden ist, der am Kopfstück (3) auf dessen dem Ohrtrichter (4) abgewandten Seite mit einer integrierten Lupe (5) zur vergrößerten Betrachtung des Gehörgangs abgeschlossen ist, wobei dem optischen Kanal (11) ein Leuchtmittel (10) zur Ausleuchtung des Gehörgangs zugeordnet ist und das Kopfstück (3) mit dem Griffstück (2) des Otoskops (1) lösbar derart verbunden ist dass das Griffstück (2) des Otoskops (1) mit einem Adapterstück (23) zum Aufsatz Otoskop-Kopfstücks (30) und/oder eines Taschenlampenkopfs versehen ist,

    **dadurch gekennzeichnet, dass**
    das Leuchtmittel wenigstens eine Leuchtdiode (10), vorzugsweise eine weiße Leuchtdiode, ist, und das Kopfstück (3) mit dem Griffstück (2) des Otoskops (1) gelenkig verbunden ist.

2.  Otoskop nach Anspruch 1, **dadurch gekennzeichnet, daß** die Leuchtdiode (10) ein zumindest annähernd tageslichtähnliches Lichtspektrum aufweist.

3.  Otoskop nach Anspruch 2, **dadurch gekennzeichnet, daß** das Griffstück (2) des Otoskops derart als Ladegriff (2) ausgebildet ist, daß innerhalb des Griffstücks (2) wenigstens eine Batterie (12) oder wenigstens ein wiederaufladbarer Akku zur Spannungsversorgung der dem optischen Kanal (11) zugeordneten Leuchtdiode (10) angeordnet ist.

4.  Otoskop nach Anspruch 3, **dadurch gekennzeichnet, daß** der im Griffstück (2) angeordnete Akku und die Leuchtdiode (10) derart aufeinander abgestimmt sind, daß die Schwellspannung $U_S$ der Leuchtdiode (10) größer, gleich einer Entladeschlußspannung ($U_0$) des jeweiligen Akkus ist.

5.  Otoskop nach Anspruch 3 oder 4, **dadurch gekennzeichnet, daß** der Akku mehrere Zellen aufweist, wobei die Zahl (z) der Akkuzellen, die Entladeschlußspannung ($U_0$) dieser Akkuzellen und die Schwellspannung ($U_S$) der Leuchtdiode (10) so gewählt sind, daß jeweils gilt

$$U_S \geq U_0 \times z.$$

6.  Otoskop nach Anspruch 5, **dadurch gekennzeich-**

**net, daß** es sich bei dem oder den Akkuzellen (12) jeweils um Nickel-Metall-Hydrid-Akkus handelt.

7. Otoskop nach einem der vorhergehenden Ansprüche, **dadurch gekennzeichnet, daß** die Leuchtdiode (10) derart innerhalb des optischen Kanals (11) angeordnet ist, daß der Abstand der Leuchtdiode (10) von der Einführspitze (6) des Ohrtrichters (4) so gewählt ist, daß sich bei bestimmungsgemäßem Gebrauch des Otoskops (1) eine gleichmäßige Ausleuchtung des untersuchenden Gehörgangs ergibt.

8. Otoskop nach Anspruch 10, dadruch **gekennzeichnet**, daß das Ladegriffstück (2) mit einem Steckkontakt (25), insbesondere zum Anschluß an eine Haushalts-Steckdose, versehen ist.

**Claims**

1. Otoscope, preferably for medical investigation of the human auditory canal, with a grip piece (2) and a head piece (3), wherein the head piece (3) is connected with an ear funnel (4) for partial introduction into an auditory canal with inclusion of an optical channel (11), which is connected, at the head piece (3) on the side thereof remote from the ear funnel (4), with an integrated magnifying glass (5) for magnified observation of the auditory canal, wherein a lighting means (10) for illumination of the auditory canal is associated with the optical channel (11) and the head piece (3) is detachably connected with the grip piece (2) of the otoscope (1) in such a manner that the grip piece (2) of the otoscope (1) is provided with an adapter (23) for attachment of the otoscope head piece (30) and/or a torch head, **characterised in that** the lighting means is at least one light-emitting diode (10), preferably a white light-emitting diode, and the head piece (3) is pivotably connected with the grip piece (2) of the otoscope (1).

2. Otoscope according to claim 1, **characterised in that** the light-emitting diode (10) has a light spectrum at least approximately similar to daylight.

3. Otoscope according to claim 2, **characterised in that** the grip piece (2) of the otoscope is constructed as a charging grip (2) in such a manner that at least one battery (12) or at least one rechargeable battery for voltage supply of the light-emitting diode (10) associated with the optical channel (11) is arranged within the grip piece (2).

4. Otoscope according to claim 3, **characterised in that** the battery arranged in the grip piece (2) and the light-emitting diode (10) are matched to one another in such a manner that the threshold voltage $U_S$ of the light-emitting diode (10) is greater than or equal to a discharge end voltage ($U_0$) of the respective battery.

5. Otoscope according to claim 3 or 4, **characterised in that** the battery has several cells, wherein the number (z) of the battery cells, the discharge voltage ($U_0$) of those battery cells and the threshold voltage ($U_S$) of the light-emitting diode (10) is so selected that in each instance

$$U_S \geq U_0 \times z.$$

6. Otoscope according to claim 5, **characterised in that** the or each battery cell (12) is a nickel-metal-hydride battery.

7. Otoscope according to one of the preceding claims, **characterised in that** the light-emitting diode (10) is arranged within the optical channel (11) in such a manner that the spacing of the light-emitting diode (10) from the insertion tip (6) of the ear funnel (4) is so selected that in the case of use of the otoscope (1) in accordance with intention a uniform illumination of the auditory canal to be investigated results.

8. Otoscope according to claim 10, **characterised in that** the charging grip piece (2) is provided with a plug contact (25), particularly for connection with a household socket.

**Revendications**

1. Otoscope, de préférence pour l'examen médical du conduit auditif humain, comportant une poignée (2) et une tête (3), la tête (3), y compris un canal optique (11), étant reliée à un spéculum auriculaire (4) destiné à être introduit partiellement dans un conduit auditif, lequel canal optique, sur le côté de la tête (3) opposé au spéculum auriculaire (4), est fermé par une loupe (5) intégrée destinée à agrandir l'observation du conduit auditif, un moyen d'éclairage (10) destiné à éclairer le conduit auditif étant associé au canal optique (11) et la tête (3) étant assemblée de manière amovible à la poignée (2) de l'otoscope (1), de telle sorte que la poignée (2) de l'otoscope (1) est munie d'un adaptateur (23) pour le montage d'une tête d'otoscope (30) et/ou d'une tête de lampe de poche, **caractérisé en ce que** le moyen d'éclairage est au moins une diode électroluminescente (10), de préférence une diode électroluminescente blanche, et la tête (3) est assemblée de manière articulée à la poignée (2) de l'otoscope (1).

**2.** Otoscope selon la revendication 1, **caractérisé en ce que** la diode électroluminescente (10) possède un spectre de lumière au moins à peu près similaire à la lumière du jour.

**3.** Otoscope selon la revendication 2, **caractérisé en ce que** la poignée (2) de l'otoscope est réalisée sous forme de poignée à pile (2) de telle sorte qu'au moins une pile (12) ou au moins un accumulateur rechargeable, destiné à alimenter en tension la diode électroluminescente (10) associée au canal optique (11), est agencé à l'intérieur de la poignée (2).

**4.** Otoscope selon la revendication 3, **caractérisé en ce que** l'accumulateur agencé dans la poignée (2) et la diode électroluminescente (10) sont ajustées l'une à l'autre de telle sorte que la tension seuil ($U_S$) de la diode électroluminescente (10) est supérieure, égale à une tension finale de décharge ($U_0$) de l'accumulateur concerné.

**5.** Otoscope selon la revendication 3 ou 4, **caractérisé en ce que** l'accumulateur comporte plusieurs cellules, le nombre (z) de cellules d'accumulateur, la tension finale de décharge ($U_0$) de ces cellules d'accumulateur et la tension seuil ($U_S$) de la diode électroluminescente (10) étant choisis de telle sorte que dans chaque cas, on applique :

$$U_s \geq U_0 \times z.$$

**6.** Otoscope selon la revendication 5, **caractérisé en ce que** la ou les cellules d'accumulateur (12) sont dans chaque cas des accumulateurs nickel-métal-hydrure.

**7.** Otoscope selon l'une quelconque des revendications précédentes, **caractérisé en ce que** la diode électroluminescente (10) est agencée à l'intérieur du canal optique (11) de telle sorte que la distance entre la diode électroluminescente (10) et la pointe d'introduction (6) du spéculum auriculaire (4) est choisie de manière à produire un éclairage uniforme du conduit auditif à examiner en cas d'utilisation de l'otoscope (1) conforme à sa destination.

**8.** Otoscope selon la revendication 10, **caractérisé en ce que** la poignée à pile (2) est munie d'un contacteur mâle (25), destiné en particulier à être raccordé à une prise de courant domestique.

FIG.1

FIG. 2

FIG. 3

FIG.4

FIG.5

FIG.6